(19) Europäisches Patentamt
European Patent Office
Office européen des brevets

(11) Veröffentlichungsnummer : **0 445 073 A1**

(12) # EUROPÄISCHE PATENTANMELDUNG

(21) Anmeldenummer : **91810110.6**

(51) Int. Cl.[5] : **C07D 405/06**, A61K 31/41

(22) Anmeldetag : **20.02.91**

(30) Priorität : **27.02.90 CH 627/90**

(43) Veröffentlichungstag der Anmeldung :
**04.09.91 Patentblatt 91/36**

(84) Benannte Vertragsstaaten :
**AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Lang, Marc, Dr.**
**Rue des Ormes 6**
**F-68170 Rixheim (FR)**
Erfinder : **Schilling, Walter, Dr.**
**Im Muspenacker**
**CH-4204 Himmelried (CH)**

(54) **Benzofurane.**

(57) Es werden Verbindungen der Formel I offengelegt,

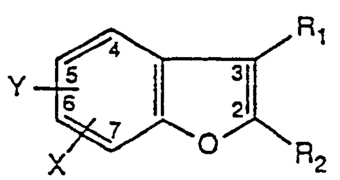

(I)

worin X beispielsweise Halogen, Cyano, Carbomoyl oder Aryloxy bedeutet sowie für andere Substituenten steht, die die in der Beschreibung angegebene Bedeutung haben, Y für eine Gruppe $-CH_2-A$ steht, in der A über ein Ringstickstoffatom gebundenes Imidazolyl, Triazolyl oder Tetrazolyl bedeutet, $R_1$ und $R_2$ unabhängig voneinander Wasserstoff, Niederalkyl oder eine Gruppe $-CH_2-A$ wie für Y definiert bedeuten, oder Y, $R_1$ und $R_2$ abhängig voneinander Bedeutungen annehmen, die in der Anmeldung beschrieben werden. Diese Verbindungen weisen wertvolle pharmazeutische Eigenschaften auf und sind insbesondere als Inhibitoren des Enzyms Aromatase und gegen Tumoren wirksam. Sie werden in bekannter Weise hergestellt.

EP 0 445 073 A1

Jouve, 18, rue Saint-Denis, 75001 PARIS

# BENZOFURANE

Die Erfindung betrifft Verbindungen der Formel I

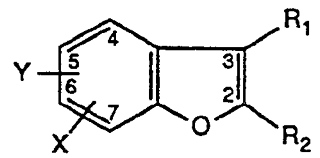

(I)

worin X Halogen, Cyano, Carbamoyl, N-Niederalkylcarbamoyl, N,N-Diniederalkylcarbamoyl, N,N-Niederalkylencarbamoyl; durch -O-, -S- oder -NR″- unterbrochenes N,N-Niederalkylencarbamoyl, worin R″ für Wasserstoff, Niederalkyl oder Niederalkanoyl steht; N-Cycloalkylcarbamoyl, N-(Niederalkyl-substituiertes Cycloalkyl)-carbamoyl, N-Cycloalkylniederalkylcarbamoyl, N-(Niederalkyl-substituiertes Cycloalkyl)-niederalkylcarbamoyl, N-Arylniederalkylcarbamoyl, N-Arylcarbamoyl, N-Hydroxycarbamoyl, Hydroxy, Niederalkoxy, Arylniederalkoxy oder Aryloxy bedeutet, Y für eine Gruppe -$CH_2$-A steht, in der A über ein Ringstickstoffatom gebundenes Imidazolyl, Triazolyl oder Tetrazolyl bedeutet, oder Y Wasserstoff bedeutet, $R_1$ und $R_2$ unabhängig voneinander Wasserstoff, Niederalkyl oder eine Gruppe -$CH_2$-A wie für Y definiert bedeuten, oder $R_1$ und $R_2$ zusammen Niederalkylen bedeuten, mit der Massgabe, dass einer der Reste Y, $R_1$ oder $R_2$ für eine Gruppe -$CH_2$-A steht, mit der weiteren Massgabe, dass in einer Gruppe -$CH_2$-A als Bedeutung von $R_1$ oder $R_2$ A von 1-Imidazolyl verschieden ist, wenn X Brom, Cyano oder Carbamoyl bedeutet, und mit der Massgabe, dass in einer Gruppe -$CH_2$-A als Bedeutung von Y A von 1-Imidazolyl verschieden ist, wenn X Halogen oder Niederalkoxy bedeutet, $R_1$ für Wasserstoff steht und $R_2$ Wasserstoff oder Niederalkyl bedeutet, und Salze davon, Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers oder zur Herstellung pharmazeutischer Präparate.

Die vor- und nachstehend verwendeten Allgemeinbegriffe haben im Rahmen der vorliegenden Anmeldung vorzugsweise die folgenden Bedeutungen:

Das Präfix "Nieder" bezeichnet einen Rest bis und mit 7 und insbesondere bis und mit 4 Kohlenstoffatomen.

Niederalkyl ist z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl.

Halogen steht insbesondere für Chlor und vor allem für Brom, kann aber auch Fluor oder Iod bedeuten.

Halogenniederalkyl ist z.B. Trifluormethyl.

Aryl ist z.B. Phenyl oder Naphthyl, wie 1- oder 2-Naphthyl. Die Phenyl- und Naphthylreste können unsubstituiert oder substituiert sein, insbesondere wie nachfolgend für Phenyl angegeben. Aryl ist bevorzugt Phenyl, das unsubstituiert oder durch 1 bis 4, vor allem 1 oder 2, Substituenten aus der Gruppe bestehend aus Niederalkyl, Niederalkenyl, Niederalkinyl, Niederalkylen (angeknüpft an zwei benachbarten C-Atomen), $C_3$-$C_8$-Cycloalkyl, Phenylniederalkyl, Phenyl, Halogenniederalkyl, Hydroxy, Niederalkoxy, Halogenniederalkoxy, Phenylniederalkoxy, Phenyloxy, Niederalkenyloxy, Halogenniederalkenyloxy, Niederalkinyloxy, Niederalkylendioxy (angeknüpft an zwei benachbarten C-Atomen), Niederalkanoyloxy, Phenylniederalkanoyloxy, Phenylcarbonyloxy, Mercapto, Niederalkylthio, Phenylniederalkylthio, Phenylthio, Niederalkylsulfinyl, Phenylniederalkylsulfinyl, Phenylsulfinyl, Niederalkylsulfonyl, Phenylniederalkylsulfonyl, Phenylsulfonyl, Halogen, Nitro, Amino, Niederalkylamino, $C_3$-$C_8$-Cycloalkylamino, Phenylniederalkylamino, Phenylamino, Diniederalkylamino, N-Niederalkyl-N-phenylamino, N-Niederalkyl-N-phenylniederalkylamino; Niederalkylenamino oder durch -O-, -S- oder -NR″ unterbrochenes Niederalkylenamino (worin R″ für Wasserstoff, Niederalkyl oder Niederalkanoyl steht); Niederalkanoylamino, Phenylniederalkanoylamino, Phenylcarbonylamino, Niederalkanoyl, Phenylniederalkanoyl, Phenylcarbonyl, Carboxy, Niederalkoxycarbonyl, Carbamoyl, N-Niederalkylcarbamoyl, N,N-Diniederalkylcarbamoyl, N,N-Niederalkylencarbamoyl; durch -O-, -S- oder -NR″- unterbrochenes N,N-Niederalkylencarbamoyl, worin R″ für Wasserstoff, Niederalkyl oder Niederalkanoyl steht; N-Hydroxycarbamoyl, N-Phenylniederalkylcarbamoyl, N-Phenylcarbamoyl, Cyano, Sulfo, Niederalkoxysulfonyl, Sulfamoyl, N-Niederalkylsulfamoyl, N,N-Diniederalkylsulfamoyl oder N-Phenylsulfamoyl substituiert sind; wobei die in den Substituenten vorkommenden Phenylgruppen ihrerseits jeweils unsubstituiert oder durch Niederalkyl, Niederalkoxy, Hydroxy, Halogen und/oder Trifluormethyl substituiert sind.

Aryl bedeutet in erster Linie Phenyl, das unsubstituiert oder durch Niederalkyl, Niederalkoxy, Hydroxy, Halogen und/oder Trifluormethyl substituiert ist, und vor allen Dingen Phenyl.

Substituiertes Phenyl ist bevorzugt disubstituiert und insbesondere monosubstituiert.

Arylniederalkoxy ist z.B. Phenylniederalkoxy und insbesondere Benzyloxy.

N-Arylcarbamoyl ist z.B. N-Phenylcarbamoyl.

Ueber ein Ringstickstoffatom gebundenes Imidazolyl ist z.B. 1-Imidazolyl.

Ueber ein Ringstickstoffatom gebundenes Triazolyl ist z.B. 1-(1,2,4-Triazolyl), 1-(1,3,4-Triazolyl), 1-(1,2,3-Triazolyl) oder 1-(1,2,5-Triazolyl).

Ueber ein Ringstickstoffatom gebundenes Tetrazolyl ist z.B. 1-Tetrazolyl oder 2-Tetrazolyl.

Niederalkylen gebildet aus den Gruppen $R_1$ und $R_2$ ist bevorzugt ein Rest $-(CH_2)_n-$, worin n für 3, 4 oder 5, insbesondere für 3 oder 4 steht, z.B. 1,3-Propylen oder vor allem 1,4-Butylen, kann aber auch, z.B. durch Niederalkyl, substituiert sein.

Cycloalkyl ist vorzugsweise $C_3$-$C_8$- und insbesondere $C_3$- oder $C_5$-$C_6$-Cycloalkyl, was bedeuten soll, dass es 3 bis 8 bzw. 3, 5 oder 6 Ringkohlenstoffatome enthält.

Niederalkylen, angeknüpft an zwei benachbarten C-Atomen eines Benzolrings, ist vorzugsweise $C_3$-$C_4$-Alkylen, z.B. 1,3-Propylen oder 1,4-Butylen.

Niederalkylendioxy, angeknüpft an zwei benachbarten C-Atomen, bedeutet vorzugsweise $C_1$-$C_2$-Alkylendioxy, z.B. Methylen- oder 1,2-Ethylendioxy.

Niederalkylenamino ist z.B. $C_4$-$C_7$- und insbesondere $C_4$-$C_5$-Alkylenamino, z.B. Piperidino. Durch -O-, -S- oder -NR''- unterbrochenes Niederalkylenamino steht z.B. für solches $C_4$-$C_7$- und insbesondere $C_4$-$C_5$-Alkylenamino, bei dem ein Ringkohlenstoffatom durch die entsprechende Heterogruppe ersetzt ist, und ist insbesondere Morpholino, Thiomorpholino, Piperazino oder 4-Niederalkyl- oder 4-Acylpiperazino. Carbamoyl bedeutet die Gruppe $-CONH_2$. Folglich steht beispielsweise N,N-Niederalkylencarbamoyl für Niederalkylenamino-carbonyl, worin Niederalkylenamino wie oben definiert ist.

Salze von erfindungsgemässen Verbindungen sind in erster Linie pharmazeutisch verwendbare, nicht-toxische Salze. Beispielsweise können Verbindungen der Formel I mit basischen Gruppen Säureadditionssalze bilden, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure oder Methansulfonsäure, oder mit Aminosäuren, wie Arginin oder Lysin. Verbindungen der Formel I mit einer sauren Gruppe, z.B. 1-Tetrazolyl, bilden z.B. Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wie Niederalkylaminen, z.B. Triethylamin, Hydroxyniederalkylaminen, z.B. 2-Hydroxyethylamin, Bis-(2-hydroxyethyl)-amin oder Tris-(2-hydroxyethyl)-amin, basischen aliphatischen Estern von Carbonsäuren, z.B. 4-Aminobenzoesäure-2-diethylaminoethylester, Niederalkylenaminen, z.B. 1-Ethylpiperidin, Cycloalkylaminen, z.B. Dicyclohexylamin, oder Benzylaminen, z.B. N,N'-Dibenzylethylendiamin, Dibenzylamin oder Benzyl-β-phenethylamin. Verbindungen der Formel I mit einer sauren und einer basischen Gruppe können auch in Form von inneren Salzen, d.h. in zwitterionischer Form, vorliegen.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch verwendbaren, nichttoxischen Salze, die deshalb bevorzugt sind.

Die erfindungsgemässen Verbindungen der Formel I weisen wertvolle, insbesondere pharmakologisch verwendbare, Eigenschaften auf. Insbesondere hemmen sie selektiv das Enzym Aromatase bei Säugern einschliesslich Menschen. Dadurch wird die metabolische Umwandlung von Androgenen zu Oestrogenen gehemmt. Die Verbindungen der Formel I sind daher z.B. zur Behandlung Oestrogen-abhängiger Krankheiten, einschliesslich Oestrogenabhängigem Brustkrebs, insbesondere bei postmenopausalen Frauen, geeignet. Ferner sind sie z.B. nützlich bei der Behandlung von Gynäkomastie, d.h. männlicher Brustentwicklung, weil die Aromatisierung der Steroide gehemmt wird.

Diese Wirkungen können durch in vitro-Versuche oder in vivo-Versuche, vorzugsweise an Säugetieren, z.B. Meerschweinchen, Mäusen, Ratten, Katzen, Hunden oder Affen, nachgewiesen werden. Die angewandte Dosierung liegt z.B. in einem Bereich von etwa 0,001 und 10 mg/kg, vorzugsweise zwischen 0,001 und 1 mg/kg.

Die in vitro-Hemmung der Aromataseaktivität kann z.B. mit der Methode, die in J. Biol. Chem. 249, 5364 (1974) beschrieben ist, gezeigt werden. Ferner können $IC_{50}$-Werte für die Aromatasehemmung z.B. in vitro aus enzymkinetischen Studien, die die Hemmung der Umwandlung von 4-$^{14}$C-Androstendion zu 4-$^{14}$C-Oestron in menschlichen plazentalen Mikrosomen betreffen, erhalten werden. Die $IC_{50}$-Werte der erfindungsgemässen Verbindungen liegen minimal etwa bei $10^{-9}$ M.

In vivo kann die Aromatasehemmung z.B. durch die Unterdrückung des ovarialen Oestrogengehalts weiblicher Ratten gezeigt werden, die zunächst mit Stutenserumgonadotropin und 2 Tage später mit menschlichem Choriongonadotropin injiziert werden, am folgenden Tag p.o. mit einer Verbindung der Erfindung behandelt werden und 1 h später mit Androstendion. Eine weitere Möglichkeit zur in vivo Bestimmung der Aromatasehemmung wird im folgenden beschrieben: Androstendion (30 mg/kg subkutan) wird allein bzw. zusammen mit einer Verbindung der Erfindung (oral oder subkutan) 4 Tage lang an nicht geschlechtsreife weibliche Ratten

verabreicht. Nach der vierten Anwendung werden die Ratten getötet, die Uteri isoliert und gewogen. Die Aromatasehemmung wird bestimmt durch das Ausmass, in dem die durch die Verabreichung von Androstendion allein verusachte Uterushypertrophie durch die gleichzeitige Verabreichung der erfindungsgemässen Verbindung unterdrückt bzw. verringert wird. Die minimale effektive Dosis der erfindungsgemässen Verbindungen bei den in vivo Tests liegt etwa zwischen 0,01 und 1 mg/kg.

Die Antitumorwirkung, insbesondere bei Oestrogen-abhängigen Tumoren, kann in vivo z.B. bei DMBA-induzierten Mammatumoren an weiblichen Sprague-Dawley-Ratten gezeigt werden [vgl. Proc. Soc. Exp. Biol. Med. 160, 296-301 (1979)]. Die Anwendung von erfindungsgemässen Verbindungen bewirkt eine Regression der Tumoren und unterdrückt weiterhin das Auftreten neuer Tumoren bei täglichen Dosen ab etwa 1 mg/kg p.o.

Darüberhinaus haben die Verbindungen der Formel I keine Hemmwirkung auf die Spaltung der Cholesterin-Seitenkette und induzieren keine adrenale Hypertrophie, was durch endokrine Organuntersuchungen gezeigt wird.

Aufgrund ihrer pharmakologischen Eigenschaften als äusserst selektive Inhibitoren des Enzyms Aromatase sind die Verbindungen der Formel I z.B. zur Behandlung Oestrogenabhängiger Krankheiten, wie Brusttumoren (Brustkarzinoma), Endometriosis, vorzeitigen Wehen oder endometrialen Tumoren bei Frauen oder Gynäkomastie bei Männern, geeignet.

Insbesondere betrifft die Erfindung die Verbindungen der Formel I, worin X Halogen, Cyano, Carbamoyl, N-Niederalkylcarbamoyl, N-Cycloalkylniederalkylcarbamoyl, N,N-Diniederalkylcarbamoyl, N-Arylcarbamoyl, Hydroxy, Niederalkoxy, Arylniederalkoxy oder Aryloxy bedeutet, wobei Aryl für Phenyl oder Naphthyl, welches jeweils unsubstituiert oder durch Niederalkyl, Hydroxy, Niederalkoxy, Halogen und/oder Trifluormethyl substituiert ist, steht; Y für eine Gruppe $-CH_2$-A steht, in der A 1-Imidazolyl, 1-(1,2,4-Triazolyl), 1-(1,3,4-Triazolyl), 1-(1,2,3-Triazolyl), 1-(1,2,5-Triazolyl), 1-Tetrazolyl oder 2-Tetrazolyl bedeutet, oder Y Wasserstoff bedeutet, $R_1$ und $R_2$ unabhängig voneinander Wasserstoff, Niederalkyl oder eine Gruppe $-CH_2$-A wie für Y definiert bedeuten, oder $R_1$ und $R_2$ zusammen $-(CH_2)_n-$, worin n für 3, 4 oder 5 steht, bedeuten, mit der Massgabe, dass einer der Reste Y, $R_1$ oder $R_2$ für eine Gruppe $-CH_2$-A steht, mit der weiteren Massgabe, dass in einer Gruppe $-CH_2$-A als Bedeutung von $R_1$ oder $R_2$ A von 1-Imidazolyl verschieden ist, wenn X Brom, Cyano oder Carbamoyl bedeutet, und mit der Massgabe, dass in einer Gruppe $-CH_2$-A als Bedeutung von Y A von 1-Imidazolyl verschieden ist, wenn X Halogen oder Niederalkoxy bedeutet, $R_1$ für Wasserstoff steht und $R_2$ Wasserstoff oder Niederalkyl bedeutet, und Salze davon.

Bevorzugt betrifft die Erfindung die Verbindungen der Formel I, worin der Rest X in 5-oder 7-Stellung angeknüpft ist und Halogen, Cyano, Carbamoyl oder Phenyloxy bedeutet; der Rest Y in 4- oder 5-Stellung angeknüpft ist und für eine Gruppe $-CH_2$-A steht, in der A 1-Imidazolyl, 1-(1,2,4-Triazolyl), 1-(1,2,3-Triazolyl), 1-Tetrazolyl oder 2-Tetrazolyl bedeutet, oder der Rest Y Wasserstoff bedeutet; $R_1$ Wasserstoff, Niederalkyl oder eine Gruppe $-CH_2$-A wie für Y definiert bedeutet, $R_2$ Wasserstoff oder Niederalkyl bedeutet, oder $R_1$ und $R_2$ zusammen $-(CH_2)_4-$ bedeuten; mit der Massgabe, dass einer der Reste Y oder $R_1$ für eine Gruppe $-CH_2$-A steht; mit der weiteren Massgabe, dass in einer Gruppe $-CH_2$-A als Bedeutung von $R_1$ A von 1-Imidazolyl verschieden ist, wenn X Brom, Cyano oder Carbamoyl bedeutet, und mit der Massgabe, dass in einer Gruppe $-CH_2$-A als Bedeutung von Y A von 1-Imidazolyl verschieden ist, wenn X Halogen bedeutet und $R_1$ für Wasserstoff steht; und Salze davon.

Hervorzuheben sind die Verbindungen der Formel I, worin der Rest X in 5- oder 7-Stellung angeknüpft ist und Halogen, Cyano, Carbamoyl oder Phenyloxy bedeutet; der Rest Y in 4- oder 5-Stellung angeknüpft ist und für eine Gruppe $-CH_2$-A steht, in der A 1-(1,2,4-Triazolyl), 1-(1,2,3-Triazolyl), 1-Tetrazolyl oder 2-Tetrazolyl bedeutet, oder der Rest Y Wasserstoff bedeutet; $R_1$ Wasserstoff, Niederalkyl oder eine Gruppe $-CH_2$-A wie für Y definiert bedeutet, $R_2$ Wasserstoff oder Niederalkyl bedeutet, oder $R_1$ und $R_2$ zusammen $-(CH_2)_4-$ bedeuten; mit der Massgabe, dass einer der Reste Y oder $R_1$ für eine Gruppe $-CH_2$-A steht; und Salze davon.

Besonders bevorzugt betrifft die Erfindung Verbindungen der Formel I, worin der Rest X in 5- oder 7-Stellung angeknüpft ist und Halogen, Cyano, Carbamoyl oder Phenyloxy bedeutet; der Rest Y in 4- oder 5-Stellung angeknüpft ist und für eine Gruppe $-CH_2$-A steht, in der A 1-Imidazolyl, 1-(1,2,4-Triazolyl), 1-(1,2,3-Triazolyl), 1-Tetrazolyl oder 2-Tetrazolyl bedeutet; $R_1$ und $R_2$ unabhängig voneinander Wasserstoff oder Niederalkyl bedeuten, oder $R_1$ und $R_2$ zusammen für $-(CH_2)_4-$ stehen; mit der Massgabe, dass in einer Gruppe Y = $-CH_2$-A A von 1-Imidazolyl verschieden ist, wenn X Halogen bedeutet und $R_1$ für Wasserstoff steht; und Salze davon.

Ferner bevorzugt sind die Verbindungen der Formel I, worin der Rest X in 5- oder 7-Stellung angeknüpft ist und Halogen, Cyano, Carbamoyl oder Phenyloxy bedeutet; der Rest Y Wasserstoff bedeutet; $R_1$ für eine Gruppe $-CH_2$-A steht, in der A 1-Imidazolyl, 1-(1,2,4-Triazolyl), 1-(1,2,3-Triazolyl), 1-Tetrazolyl oder 2-Tetrazolyl bedeutet, $R_2$ Wasserstoff oder Niederalkyl bedeutet, mit der Massgabe, dass in einer Gruppe $R_1$ = $-CH_2$-A A von 1-Imidazolyl verschieden ist, wenn X Brom, Cyano oder Carbamoyl bedeutet; und Salze davon.

In erster Linie bevorzugt sind die Verbindungen der Formel I, worin der Rest X in 7-Stellung angeknüpft

ist und Brom, Cyano, Carbamoyl oder Phenyloxy bedeutet; der Rest Y in 4-Stellung angeknüpft ist und für eine Gruppe $-CH_2-A$ steht, in der A 1-Imidazolyl, 1-(1,2,4-Triazolyl), 1-Tetrazolyl oder 2-Tetrazolyl bedeutet; $R_1$ und $R_2$ unabhängig voneinander Niederalkyl bedeuten, oder $R_1$ und $R_2$ zusammen für $-(CH_2)_4-$ stehen; und Salze davon.

Hervorzuheben sind ferner die Verbindungen der Formel I, worin X Halogen, Cyano, Carbamoyl, Hydroxy, Niederalkoxy oder Phenyloxy, worin Phenyl unsubstituiert oder durch Niederalkyl, Hydroxy, Niederalkoxy, Halogen und/oder Trifluormethyl substituiert ist, bedeutet, Y für eine Gruppe $-CH_2-A$ steht, in der A 1-Imidazolyl, 1-(1,2,4-Triazolyl), 1-(1,3,4-Triazolyl), 1-(1,2,3-Triazolyl), 1-(1,2,5-Triazolyl), 1-Tetrazolyl oder 2-Tetrazolyl bedeutet, $R_1$ Niederalkyl bedeutet, $R_2$ Wasserstoff oder Niederalkyl bedeutet, oder $R_1$ und $R_2$ zusammen $-(CH_2)_n-$, worin n für 3 oder 4 steht, bedeuten, und Salze davon.

Besonders hervorzuheben sind die Verbindungen der Formel I, worin der Rest X in 4-oder 7-Stellung angeknüpft ist und Halogen, Cyano, Carbamoyl oder Phenyloxy bedeutet; der Rest Y in 4- oder 5-Stellung angeknüpft ist und für eine Gruppe $-CH_2-A$ steht, in der A 1-Imidazolyl, 1-(1,2,4-Triazolyl), 1-(1,2,3-Triazolyl), 1-Tetrazolyl oder 2-Tetrazolyl bedeutet, $R_1$ Niederalkyl bedeutet, $R_2$ Wasserstoff oder Niederalkyl bedeutet, oder $R_1$ und $R_2$ zusammen $-(CH_2)_4-$ bedeuten; und Salze davon.

Als Untergruppen aus einer Gruppe von Verbindungen der Formel I sind jeweils hervorzuheben: (a) Verbindungen der Formel I, worin der Rest X in 7-Stellung und der Rest Y in 4-Stellung angeknüpft ist; (b) Verbindungen der Formel I, worin X Brom oder Cyano bedeutet; (c) Verbindungen der Formel I, worin X Carbamoyl bedeutet; (d) Verbindungen der Formel I, worin Y für eine Gruppe $-CH_2-A$ steht, in der A 1-Imidazolyl oder 1-(1,2,4-Triazolyl) bedeutet.

Die Erfindung betrifft vor allem die in den Beispielen beschriebenen spezifischen Verbindungen und pharmazeutisch verwendbare Salze davon.

Die Verbindungen der Formel I können in an sich bekannter Weise hergestellt werden, indem man z.B.

(a) ein reaktives verestertes Derivat einer Hydroxymethyl-Verbindung der Formel II

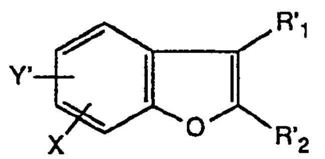

(II)

worin einer der Reste Y', $R'_1$ oder $R'_2$ Hydroxymethyl bedeutet und die beiden anderen Reste jeweils wie Y, $R_1$ bzw. $R_2$ unter Formel I definiert sind, und X wie unter Formel I definiert ist, mit einer Verbindung

A - H (III)

worin A wie unter Formel I definiert ist, oder einem N-geschützten Derivat davon, kondensiert, oder

(b) in einer Verbindung der Formel IV

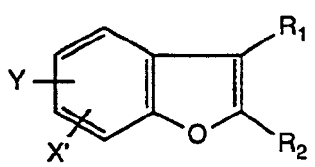

(IV)

worin X' einen in eine Gruppe X überführbaren Rest bedeutet und Y, $R_1$ und $R_2$ wie unter Formel I definiert sind, den Rest X' in eine Gruppe X überführt, oder

(c) zur Herstellung von Verbindungen der Formel I, worin A in der Gruppe $-CH_2-A$ 1-Tetrazolyl bedeutet, eine Verbindung der Formel V

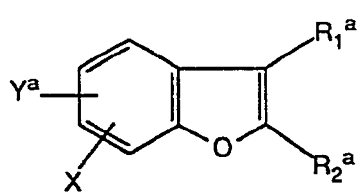

(V)

worin einer der Reste $Y^a$, $R_1^a$ oder $R_2^a$ Isocyanomethyl bedeutet und die beiden anderen Reste jeweils wie Y, $R_1$ bzw. $R_2$ unter Formel I definiert sind, und X wie unter Formel I definiert ist, mit Stickstoffwasserstoffsäure oder insbesondere einem Salz davon umsetzt; und/oder, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, und/oder ein erhaltenes Gemisch von isomeren Verbindungen der Formel I in die einzelnen Isomeren auftrennt.

In der folgenden näheren Beschreibung der Verfahren a), b) und c) haben die Symbole X, Y, A, $R_1$ und $R_2$ jeweils die unter Formel I angegebene Bedeutung, sofern nichts anderes angegeben ist.

Verfahren (a):

In einer Verbindung der Formel II steht reaktives verestertes Hydroxymethyl für Hydroxymethyl, das durch eine Abgangsgruppe verestert ist, z.B. Niederalkyloder Aryl-sulfonyloxymethyl, wie Methylsulfonyloxymethyl oder p-Toluolsulfonyloxymethyl, oder Halogenmethyl, z.B. Chlormethyl, Brommethyl oder Iodmethyl.

Setzt man bei der Umsetzung gemäss Verfahren (a) 1,2,4-Triazol als Verbindung der Formel III ein, so erhält man - abhängig von den gewählten Reaktionsbedingungen-normalerweise Gemische von Verbindungen der Formel I, worin A 1-(1,2,4-Triazolyl) bzw. 1-(1,3,4-Triazolyl) bedeutet, die sich z.B. chromatographisch auftrennen lassen. Setzt man entsprechend 1,2,3-Triazol als Verbindung der Formel III ein, so erhält man normalerweise Gemische von Verbindungen der Formel I, worin A 1-(1,2,3-Triazolyl) bzw. 1-(1,2,5-Triazolyl) bedeutet, die sich ebenfalls z.B. chromatographisch auftrennen lassen. Setzt man entsprechend Tetrazol als Verbindung der Formel III ein, so erhält man normalerweise Gemische von Verbindungen der Formel I, worin A 1-Tetrazolyl bzw. 2-Tetrazolyl bedeutet, die sich ebenfalls leicht z.B. durch Chromatographie auftrennen lassen. In einigen Fällen ist es möglich, durch die Verwendung von Verbindungen der Formel III, worin ein bestimmtes Ringstickstoffatom durch eine Schutzgruppe geschützt ist, selektiv nur eine der jeweils in Frage kommenden zwei Verbindungen zu erhalten.

Geeignete Schutzgruppen für ein Ringstickstoffatom in einer Verbindung der Formel III sind z.B. Triniederalkylsilyl, z.B. Trimethylsilyl, Niederalkanoyl, z.B. Acetyl, N,N-Diniederalkylcarbamoyl, z.B. N,N-Dimethylcarbamoyl, oder Triarylmethyl, z.B. Triphenylmethyl.

Eine weitere geeignete Schutzgruppe ist Amino bzw. Ammonium, die insbesondere bei der selektiven Herstellung von 1-(1,2,4-Triazolyl)-Verbindungen nützlich ist. Zu diesem Zweck setzt man bei der Umsetzung gemäss Verfahren (a) 4H-1,2,4-Triazol-4-amin (= 1-Amino-1,3,4-triazol) als Verbindung der Formel III ein. Man erhält zunächst eine quaternäre 1-Benzofuranylmethyl-4-amino-1,2,4-triazoliumverbindung, die z.B. durch Behandlung mit Salzsäure, 50 % unterphosphorige Säure ($H_3PO_2$) und Natriumnitrit in die gewünschte 1-Benzofuranylmethyl-1,2,4-triazolylverbindung der Formel I überführt wird.

Die Kondensationsreaktion gemäss Verfahren (a) ist an sich bekannt und entspricht einer gewöhnlichen N-Alkylierung, die z.B. ohne Zusatz von Basen oder vorzugsweise in Gegenwart von Basen, wie z.B. Kaliumcarbonat, Natrium, Triethylamin oder Pyridin, durchgeführt wird.

Die Ausgangsverbindungen der Formel II werden bevorzugt in an sich bekannter Weise aus den entsprechenden Hydroxymethyl-Verbindungen durch Veresterung erhalten. Die Hydroxymethyl-Verbindungen sind z.B. durch Reduktion, z.B. mit $LiAlH_4$ oder Diisobutylaluminiumhydrid, aus den entsprechenden Carboxy- oder Niederalkoxycarbonylverbindungen erhältlich. Letztere sind an sich bekannt oder können analog zu bekannten substituierten Benzofurancarbonsäuren bzw. -estern (vgl. auch die Beispiele 1 und 6) hergestellt werden.

Verfahren (b):

Reste X', die in eine Gruppe X überführbar sind, sind z.B. Amino, das z.B. via Diazotierung beispielsweise in Halogen, Cyano oder Hydroxy umgewandelt werden kann, oder Carboxy, Niederalkoxycarbonyl, Halogencarbonyl, z.B. -COCl, oder ein Säureanhydrid, welche durch Umsetzung mit Ammoniak bzw. dem entsprechenden primären oder sekundären Amin in Carbamoyl bzw. N-mono- oder N,N-disubstituiertes Carbamoyl überführt werden können. Die Umwandlung von Substituenten an aromatischen Systemen gemäss Verfahren (b) ist an sich bekannt.

Die Ausgangsverbindungen der Formel IV werden z.B. analog Verfahren (a) hergestellt, wobei in den entsprechenden Umsetzungen anstelle des Restes X ein Rest X' eingesetzt wird.

Verfahren (c):

Isocyanomethyl bedeutet einen Rest $-CH_2-N=C$. Salze der Stickstoffwasserstoffsäure sind insbesondere Alkalimetallazide, z.B. Natriumazid.

Die Ausgangsverbindungen der Formel V werden z.B. aus den analogen Verbindungen der Formel II, worin einer der Reste Y', $R'_1$ oder $R'_2$ z.B. Brommethyl bedeutet, hergestellt. Letztere werden zunächst in an sich bekannter Weise, z.B. durch Umsetzung mit Hexamethylentetramin (Urotropin), in die entsprechenden Aminomethyl-Verbindungen und dann in an sich bekannter Weise, z.B. durch Umsetzung mit Dichlorcarben (z.B. aus Chloroform und konz. KOH), in die gewünschten Isocyanomethylverbindungen der Formel V überführt.

Verbindungen der Formel I können in an sich bekannter Weise in andere Verbindungen der Formel I umgewandelt werden.

Zum Beispiel können Verbindungen der Formel I, worin X Halogen, insbesondere Brom, bedeutet, durch Umsetzung mit einem Cyanierungsmittel, z.B. Kupfer(I)cyanid, in andere Verbindungen der Formel I, worin X Cyano bedeutet, überführt werden.

Ferner können z.B. Verbindungen der Formel I, worin X Halogen, insbesondere Brom, bedeutet, durch Umsetzung mit Hydroxyarylverbindungen bzw. entsprechenden Alkalimetallsalzen davon, z.B. Kaliumphenolat, in andere Verbindungen der Formel I, worin X Aryloxy bedeutet, überführt werden, vorteilhaft z.B. in Gegenwart von Kupfer.

Weiterhin können z.B. Verbindungen der Formel I, worin X Cyano bedeutet, durch partielle Hydrolyse, z.B. mit Kaliumcarbonat und wässriger $H_2O_2$-Lösung, in andere Verbindungen der Formel I, worin X Carbamoyl bedeutet, überführt werden.

Andererseits können z.B. auch Verbindungen der Formel I, worin X Carbamoyl bzw. N-Niederalkylcarbamoyl bedeutet, unter Wasser- bzw. Niederalkanolabspaltung in Verbindungen der Formel I, worin X Cyano bedeutet, überführt werden.

Schliesslich können Verbindungen der Formel I, worin X Cyano bedeutet, auch direkt in Verbindungen der Formel I, worin X z.B. N-Niederalkylcarbamoyl oder N-Cycloalkylniederalkylcarbamoyl bedeutet, überführt werden, indem man z.B. erstere zunächst mit KOH/tert.-Butanol behandelt und dann mit einem Niederalkyl- bzw. Cycloalkylniederalkylhalogenid umsetzt [S. Linke, Synthesis 1978, 303].

Verfahrensgemäss erhältliche freie Verbindungen der Formel I mit salzbildenden Eigenschaften können in an sich bekannter Weise in ihre Salze überführt werden, Verbindungen mit basischen Eigenschaften z.B. durch Behandeln mit Säuren oder geeigneten Derivaten davon, Verbindungen mit sauren Eigenschaften z.B. durch Behandeln mit Basen oder geeigneten Derivaten davon.

Erfindungsgemäss erhältliche Gemische von Isomeren können in an sich bekannter Weise in die einzelnen Isomeren aufgetrennt werden, Racemate z.B. durch Bilden von Salzen mit optisch reinen salzbildenden Reagentien und Auftrennen des so erhältlichen Diastereomerengemisches, z.B. mittels fraktionierter Kristallisation.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -70°C bis etwa 200°C, vorzugsweise von etwa -20°C bis etwa 150°C, z.B. beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

Infolge der engen Beziehung zwischen den Verbindungen der Formel I in freier Form und in Form von Salzen sind vor- und nachstehend unter den freien Verbindungen bzw. ihren Salzen sinn- und zweckmässig gegebenenfalls auch die entsprechenden Salze bzw. freien Verbindungen zu verstehen.

Die Verbindungen, einschliesslich ihrer Salze, können auch in Form von Hydraten erhalten werden, oder ihre Kristalle können z.B. das zur Kristallisation verwendete Lösungsmittel einschliessen.

Im Verfahren der vorliegenden Erfindung werden vorzugsweise solche Ausgangsstoffe eingesetzt, die zu den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon, verwendet wird.

Die vorliegende Erfindung betrifft ebenfalls pharmazeutische Präparate, die als Wirkstoff eine der pharmakologisch wirksamen Verbindungen der Formel I enthalten. Besonders bevorzugt sind Präparate zur enteralen, insbesondere oralen, sowie zur parenteralen Verabreichung. Die Präparate enthalten den Wirkstoff allein

oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Die pharmazeutischen Präparate enthalten von etwa 0,1 % bis etwa 95 % des Wirkstoffs, wobei einzeldosierte Applikationsformen vorzugsweise von etwa 1 % bis etwa 90 % und nicht-einzeldosierte Applikationsformen vorzugsweise etwa 0,1 % bis etwa 20 % Wirkstoff aufweisen. Dosiseinheitsformen, wie Dragées, Tabletten oder Kapseln, enthalten von etwa 0,5 mg bis etwa 100 mg des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zu parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls ein Verfahren zur Behandlung der oben genannten Krankheitszustände. Die Verbindungen der vorliegenden Erfindung können prophylaktisch oder therapeutisch verabreicht werden, wobei man sie vorzugsweise in Form von pharmazeutischen Präparaten verwendet. Dabei wird bei einem Körpergewicht von etwa 70 kg eine tägliche Dosis von etwa 0,5 mg bis etwa 100 mg, vorzugsweise von etwa 1 mg bis etwa 20 mg, einer Verbindung der vorliegenden Erfindung verabreicht.

Die nachfolgenden Beispiele illustrieren die vorliegende Erfindung; Temperaturen werden in Grad Celsius

angegeben. Folgende Abkürzungen werden verwendet: Ether = Diethylether, Essigester = Essigsäureethylester, THF = Tetrahydrofuran; Hexan = n-Hexan; DMF = Dimethylformamid; DMSO = Dimethylsulfoxid; DC = Dünnschichtchromatographie.

Beispiel 1: 7-Brom-4-[1-(1,2,4-triazolyl)methyl]-2,3-dimethylbenzofuran

Zu einer Lösung von 318 mg 7-Brom-4-brommethyl-2,3-dimethylbenzofuran in 10 ml abs. Aceton werden nacheinander 104 mg 1,2,4-Triazol, 139 mg Kaliumcarbonat und 10 mg Kaliumiodid gegeben. Nach 2 h Rühren bei 55° wird die Suspension auf Raumtemperatur abgekühlt und das Aceton verdampft. Das Reaktionsgemisch wird zwischen Methylenchlorid und Wasser verteilt, die organische Phase mit Sole gewaschen, über Natriumsulfat getrocknet und zur Trockne eingedampft. Das erhaltene Oel wird mit Chloroform über Kieselgel chromatographiert, wobei man die Titelverbindung erhält. Sie wird aus Methylenchlorid/Ether/Hexan umkristallisiert; Smp. 108-110°. IR ($CH_2Cl_2$): 1631, 1605, 1504, 1404, 1199, 1140 $cm^{-1}$.

Die Ausgangsverbindungen werden wie folgt hergestellt:

(a) 4-Brom-3-(butan-3-on-2-yl)oxy-benzoesäureethylester

Zu 3,08 g 4-Brom-3-hydroxybenzoesäureethylester (s. DE-A-2 062 611 ) und 1,46 g 3-Chlor-2-butanon werden 5,17 g Kaliumcarbonat in 20,5 ml Aceton gegeben. Nach 16 h Rühren unter Rückfluss wird die sandgelbe Suspension auf Raumtemperatur abgekühlt und filtriert. Das Filtrat wird bei vermindertem Druck eingedampft, wobei man die Titelverbindung als ein farbloses Oel erhält, das ohne weitere Reinigung weiterverwendet wird. DC (Kieselgel; $CH_2Cl_2$/Methanol 95:5) $R_f$ = 0,69. IR ($CH_2Cl_2$): 1720, 1590, 1575, 1480, 1415, 1290 $cm^{-1}$.

(b) 7-Brom-2,3-dimethylbenzofuran-4-carbonsäure

34 g 4-Brom-3-(butan-3-on-2-yl)oxy-benzoesäureethylester werden tropfenweise innerhalb von 20 min zu 25 ml eisgekühlter konz. Schwefelsäure gegeben. Das Gemisch wird 25 h unter Stickstoff gerührt (Badtemperatur: 50°). Das Reaktionsgemisch wird zunächst auf ein Gemisch aus Eiswasser und Essigester gegossen und dann zwischen Essigester und gesättigter wässriger Natriumhydrogencarbonatlösung verteilt. Die wässrige Phase wird abgetrennt und zweimal mit Essigester gewaschen. Die vereinigten organischen Extrakte werden nochmals mit wässriger Natriumhydrogencarbonatlösung extrahiert. Die vereinigten wässrigen Extrakte werden mit konz. Salzsäure auf pH 1 gebracht, wobei ein gelber Feststoff ausfällt, der abfiltriert wird. Nach Umkristallisation aus Ether erhält man die Titelverbindung. DC (Kieselgel; Toluol/Essigester 9:1 ) $R_f$ = 0,3. IR (DMSO-$d_6$): 1710, 1625, 1260, 1170 $cm^{-1}$.

(c) 7-Brom-4-hydroxymethyl-2,3-dimethylbenzofuran

1,5 8 g 7-Brom-2,3-dimethylbenzofuran-4-carbonsäure werden portionsweise zu einer auf 0° gekühlten Suspension von 226,3 mg Lithiumaluminiumhydrid in 18 ml abs. THF gegeben. Nach erfolgter Zugabe wird die Lösung 30 min bei 0° weitergerührt und dann 19 h bei Raumtemperatur. Dann wird das Reaktionsgemisch zwischen 1 N Salzsäure und Essigester verteilt. Die wässrige Phase wird abgetrennt und die organische Phase jeweils zweimal mit wässriger Natriumhydrogencarbonatlösung und mit Sole gewaschen. Die organische Phase wird über Natriumsulfat getrocknet und das Lösungsmittel bei vermindertem Druck abgedampft, wobei man die Titelverbindung als hellgelben Feststoff erhält. DC ($CH_2Cl_2$/Methanol 95:5) $R_f$ = 0,5. IR ($CH_2Cl_2$): 3597, 2923, 1632, 1602, 1404, 1206 $cm^{-1}$.

(d) 7-Brom-4-brommethyl-2,3-dimethylbenzofuran

1,98 g 7-Brom-4-hydroxymethyl-2,3-dimethylbenzofuran werden bei 0° innerhalb von 15 min zu einer Lösung von 0,8 ml Phosphortribromid in 20 ml abs. Ether gegeben. Nach 2 h Rühren bei 0° und weiteren 30 min bei Raumtemperatur wird das Gemisch zwischen Essigester und eisgekühltem Wasser verteilt. Die organische Phase wird nacheinander mit Wasser, wässriger Natriumhydrogencarbonatlösung und zweimal mit Sole gewaschen. Nach dem Trocknen über Natriumsulfat und Verdampfen des Lösungsmittels bei vermindertem Druck erhält man die reine Titelverbindung. DC (Kieselgel, Chloroform/Methanol 95:5) $R_f$ = 0,85. IR ($CH_2Cl_2$): 1630, 1601, 1487, 1444, 1402, 1203 $cm^{-1}$.

Beispiel 2: 7-Cyano-4-[1-(1,2,4-triazolyl)methyl]-2,3-dimethylbenzofuran

Ein Gemisch von 306,2 mg 7-Brom-4-[1-(1,2,4-triazolyl)methyl]-2,3-dimethylbenzofuran (s. Beispiel 1) und 121,8 mg Kupfer(I)cyanid in 0,8 ml Pyridin wird unter Stickstoff 18 h unter Rückfluss gekocht. Das schwarze Reaktionsgemisch wird mit Essigester und 37%-iger wässriger Ammoniaklösung behandelt und die organische Phase abgetrennt. Die organische Phase wird nochmals mit wässriger Ammoniaklösung, dann zweimal mit 0,1 N Salzsäure und schliesslich zweimal mit Wasser gewaschen, getrocknet und filtriert. Nach Verdampfen des Lösungsmittels bei vermindertem Druck erhält man die Titelverbindung. DC ($CH_2Cl_2$/Methanol 95:5) $R_f$ = 0,38. IR ($CH_2Cl_2$): 2233, 1615, 1504, 1408 $cm^{-1}$.

Beispiel 3: 7-Brom-4-(1-imidazolylmethyl)-2,3-dimethylbenzofuran

1,76 g Natrium werden portionsweise zu einer Lösung von 22,3 g Imidazol in 350 ml abs. THF, die bei 45° gehalten wird, gegeben. Nach 2,5 h Rühren bei 45° wird das orangefarbene Reaktionsgemisch mit einer Lösung von 16,7 g 7-Brom-4-brommethyl-2,3-dimethylbenzofuran (s. Beispiel 1d) in 250 ml abs. THF behandelt. Nach 2 h wird das Lösungsmittel bei vermindertem Druck verdampft und das Reaktionsgemisch mit wässriger Natriumchloridlösung und 180 ml 1 N Natriumhydrogencarbonatlösung behandelt. Es wird dreimal mit Ether extrahiert, die Extrakte werden mit Sole gewaschen, über Natriumsulfat getrocknet und zur Trockne eingedampft, wobei man die Titelverbindung erhält. Smp. 158-160°. DC (Chloroform ethanol 9:1) $R_f$ = 0,49. IR ($CH_2Cl_2$): 1630, 1500, 1400, 1385, 1230, 1209, 1150 $cm^{-1}$.

Beispiel 4: 4-(1-Imidazolylmethyl)-7-phenoxy-2,3-dimethylbenzofuran-hydrochlorid

Ein Gemisch aus 25,4 g 7-Brom-4-(1-imidazolylmethyl)-2,3-dimethylbenzofuran (s. Beispiel 3), 11 g Kaliumphenolat und 250 mg Kupferpulver wird 18 h bei 150° gerührt. Das Reaktionsgemisch wird auf Raumtemperatur abgekühlt und über Kieselgel chromatographiert ($CH_2Cl_2$/Methanol 99,5:0,5), wobei man die Titelverbindung als freie Base erhält. Diese wird gelöst in Methanol mit etherischer HCl-Lösung behandelt, wobei man die Titelverbindung erhält; Smp. 180- 181 ° (nach Kristallisation aus Methanol/Ether); IR (Nujol): 2550, 1470, 1380, 1220 $cm^{-1}$.

Beispiel 5: 7-Cyano-4-(1 -imidazolylmethyl)-2,3-dimethylbenzofuran-hydrochlorid

Ein Gemisch von 1 g 7-Brom-4-(1-imidazolylmethyl)-2,3-dimethylbenzofuran (s. Beispiel 3) und 0,32 g Kupfer(I)cyanid in 3 ml N-Methyl-2-pyrrolidon wird 20 h bei 200° gerührt. Man kühlt das Reaktionsgemisch ab, giesst es in eine eisgekühlte 50 % wässrige Ethylendiaminlösung und extrahiert mit Methylenchlorid. Die organischen Extrakte werden jeweils zweimal mit 50%-iger wässriger Ethylendiaminlösung und Wasser gewaschen, über Natriumsulfat getrocknet und zur Trockne eingedampft, wobei man die Titelverbindung in roher Form erhält. Letztere wird über Kieselgel chromatographiert (Chloroform/Methanol 95:5), wobei man die Titelverbindung in reiner Form erhält. DC (Chloroform/Methanol 95:5) $R_f$ = 0,31. IR ($CH_2Cl_2$): 2250, 1625, 1505, 1235, 1210 $cm^{-1}$. Das Hydrochlorid der Titelverbindung wird in analoger Weise wie in Beispiel 4 beschrieben erhalten; Smp. 258-260° (Zersetzung).

Beispiel 6: 7-Brom-3-(1-imidazolylmethyl)-benzofuran-hydrochlorid

Eine Lösung von 4,35 g Imidazol in 45 ml abs. THF wird bei 20° innerhalb von 30 min mit 0,63 g Natrium versetzt. Nach 2,5 h Rühren - nachdem das gesamte Natrium verbraucht ist - versetzt man das Reaktionsgemisch innerhalb von 20 min tropfenweise mit einer Lösung von 7,9 g 7-Brom-3-brommethyl-benzofuran in 25 ml THF. Nach weiteren 1,5 h Rühren bei Raumtemperatur wird das Reaktionsgemisch mit 300 ml Wasser versetzt und zweimal mit je 300 ml Essigester extrahiert. Die vereinigten organischen Extrakte werden noch 5 x mit je 50 ml Wasser und einmal mit 70 ml Sole gewaschen, über Natriumsulfat getrocknet und eingeengt. Die Reinigung erfolgt durch Säulenchromatographie (Kieselgel/Chloroform : Methanol 9:1). Die freie Base wird in Ethanol/Ether gelöst und durch Behandlung mit 6N etherischer HCl-Lösung in die Titelverbindung überführt; Smp. 192-197° (Zersetzung). IR (Nujol): 2960, 1465, 1415, 1370, 1285, 1280 $cm^{-1}$.

Die Ausgangsverbindungen werden wie folgt hergestellt:

(a) 2-(2-Bromphenoxy)-essigsäureethylester

Zu 346 g o-Bromphenol in 1 l Aceton gibt man 245,2 g Chloressigsäure-ethylester und 552 g Pottasche. Die gelbe Suspension wird auf Rückflusstemperatur erhitzt und 24 h bei dieser Temperatur gerührt. Die Suspension wird abgenutscht und der Rückstand mehrmals mit Aceton nachgewaschen. Die Lösung wird im Hochvakuum eingeengt, das erhaltene Oel wird in Ether gelöst und nacheinander unter Eiskühlung mit 3x 150 ml 2 N NaOH und 3 x 150 ml Sole gewaschen. Die Etherphase wird über Natriumsulfat getrocknet, filtriert und eingedampft. Die reine Titelsubstanz wird durch Destillation unter vermindertem Druck gewonnen. Sdp.0,107 mbar 93-97°. DC (Kieselgel/ Chloroform): $R_f$ = 0,69.

(b) 3-(2-Bromphenoxy)-2-oxo-bernsteinsäurediethylester

Zu einer Suspension von 86,6 g 50 % Natriumhydrid in 2 l Ether werden innerhalb von 20 min 99 ml Ethanol zugetropft. Die Temperatur steigt dabei auf 25°. Die erhaltene Suspension wird tropfenweise innerhalb von 25 min mit 245 ml Oxalsäurediethylester versetzt. Das Reaktionsgemisch wird auf Rückflusstemperatur erhitzt und dann mit einer Lösung von 425,3 g 2-(2-Bromphenoxy)-essigsäureethylester in 500 ml Ether versetzt. Nach 30 min - wenn alles gelöst ist - wird das Reaktionsgemisch abgekühlt und unter Rühren auf

1,5 kg Eis gegossen. Durch Zugabe von 2 N HCl wird ein pH-Wert von 3 eingestellt und anschliessend die Phasen getrennt. Die organische Phase wird mit Wasser, dann mit Sole gewaschen und mit Natriumsulfat getrocknet. Nach Einengen unter vermindertem Druck wird die erhaltene Titelverbindung [IR ($CH_2Cl_2$): 1750, 1670, 1590 $cm^{-1}$] ohne zusätzliche Reinigung weiter umgesetzt.

(c) 7-Brom-2,3-di-(ethoxycarbonyl)-benzofuran

Zu 1, 135 l 90%-iger Schwefelsäure tropft man innerhalb von 1 h unter Rühren und bei Raumtemperatur 366,4 g 3-(2-Bromphenoxy)-2-oxo-bernsteinsäurediethylester. Das dunkelbraune Reaktionsgemisch wird anschliessend 1 Tag bei 55° gerührt. Nach Abkühlen wird unter Rühren auf 2 kg Eis gegossen. Nach Extraktion mit Ether und Abtrennen der wässrigen Schicht wird nacheinander mit Sole, 1 N wässriger Natriumhydrogencarbonatlösung und Sole neutral gewaschen. Nach Trocknen über Natriumsulfat, Filtrieren und Einengen unter vermindertem Druck wird das so erhaltene Rohprodukt durch Chromatographie (Kieselgel/Chloroform) gereinigt. DC (Chloroform) $R_f$ = 0,67. IR ($CH_2Cl_2$): 1740, 1600, 1475, 1370 $cm^{-1}$.

(d) 7-Brom-3-ethoxycarbonyl-benzofuran

Ein Gemisch von 87,7 g 7-Brom-2,3-di-(ethoxycarbonyl)-benzofuran, 30 g Natriumchlorid und 9,2 ml Wasser wird in 530 ml DMSO 3 h bei 150° gerührt ($CO_2$-Entwicklung). Nach Abkühlen des Reaktionsgemisches wird vom Ungelösten über Hyflo Super Cel® (Kieselgur) abfiltriert. Das Filtrat wird bei 40° unter vermindertem Druck vom DMSO befreit. Der Rückstand wird in Essigester gelöst und mit 3 x 300 ml Wasser und 1 x 300 ml Sole gewaschen. Die organische Phase wird über Natriumsulfat getrocknet und unter vermindertem Druck eingeengt. Der schwarze Rückstand wird durch Säulenchromatographie (Kieselgel/Chloroform) gereinigt. Die Titelverbindung wird aus Petrolether umkristallisiert; Smp. 54-56°. IR ($CH_2Cl_2$): 1725, 1590, 1585, 1480 $cm^{-1}$.

(e) 7-Brom-3-hydroxymethyl-benzofuran

182,6 ml einer 20%-igen Lösung von Diisobutylaluminiumhydrid in Toluol werden bei 0° unter Stickstoff innerhalb von 60 min mit 28 g 7-Brom-3-ethoxycarbonyl-benzofuran versetzt. Nach weiteren 60 min Rühren bei 0° wird auf ein Gemisch von 400 ml Eis und 50 ml 70 % Schwefelsäure gegossen. Nach Rühren und Abtrennen der organischen Phase wird noch dreimal mit je 50 ml Toluol extrahiert. Die vereinigten Extrakte werden mit 4 x 35 ml Wasser und 1 x 30 ml Sole gewaschen, über Natriumsulfat getrocknet und unter vermindertem Druck eingeengt. Der Rückstand wird aus Essigester/Petrolether kristallisiert; Smp. 111-112°. IR ($CH_2Cl_2$): 3600, 1615, 1595, 1470 $cm^{-1}$.

(f) 7-Brom-3-brommethyl-benzofuran

Eine Lösung von 2,73 g Phosphortribromid in 50 ml Ether wird bei 3° innerhalb von 30 min mit 6,25 g 7-Brom-3-hydroxymethyl-benzofuran versetzt. Nach Temperaturanstieg auf 15° wird noch 1 h bei 5° gerührt und anschliessend auf 100 ml Eis gegossen. Nach Zugabe von Ether werden die Phasen getrennt. Die organische Lösung wird mit 3 x 40 ml Wasser und 1 x 40 ml Sole gewaschen, über Natriumsulfat getrocknet und eingeengt. Die Titelverbindung wird aus Essigester/Petrolether kristallisiert; Smp. 123-124,5°; IR ($CH_2Cl_2$): 1590, 1480, 1415 $cm^{-1}$.

Beispiel 7: 7-Cyano-3-(1-imidazolylmethyl)-benzofuran-hydrochlorid

Analog Beispiel 5 werden 5,98 g 7-Brom-3-(1 -imidazolylmethyl)-benzofuran (s. Beispiel 6) mit 2,13 g Kupfer(I)cyanid in 19,5 ml N-Methyl-2-pyrrolidon in die Titelverbindung überführt, welche aus Ethanol/Ether kristallisiert wird; Smp. 237-239°; IR (KBr): 3090, 3000, 2800, 2236, 1575, 1425, 1280 $cm^{-1}$.

Beispiel 8: 7-Brom-5-(1-imidazolylmethyl)-2,3-dimethyl-benzofuran-hydrochlorid

Analog Beispiel 6 werden 31,8 g 7-Brom-5-brommethyl-2,3-dimethyl-benzofuran in die Titelverbindung überführt; Smp. (nach Kristallisation aus Methanol/Ether) 272-274°. IR (Nujol): 2962, 1464, 1416, 1370, 1287, 1281 $cm^{-1}$.

Die Ausgangsverbindung wird wie folgt hergestellt:

(a) 7-Brom-5-brommethyl-2,3-dimethyl-benzofuran

Analog Beispiel 1d werden 76,5 g 7-Brom-5-hydroxymethyl-2,3-dimethylbenzofuran in die Titelverbindung überführt; Smp. 122-124°. IR ($CH_2Cl_2$): 1629, 1600, 1488, 1443, 1404, 1203 $cm^{-1}$.

Beispiel 9: 7-Cyano-5-(1-imidazolylmethyl)-2,3-dimethyl-benzofuran-hydrochlorid

Analog Beispiel 5 werden 12,2 g 7-Brom-5-(1-imidazolylmethyl)-2,3-dimethyl-benzofuran (s. Beispiel 8) mit 3,9 g Kupfer(I)cyanid in die Titelverbindung überführt; Smp. (nach Kristallisation aus Ethanol/Ether) 258-259°. IR (KBr): 3092, 2238, 1470, 1285 $cm^{-1}$.

Beispiel 10: 5-Brom-3-(1-imidazolylmethyl)-benzofuran-hydrochlorid

Analog Beispiel 6 werden 33,05 g 5-Brom-3-brommethyl-benzofuran in die Titelverbindung überführt; Smp. (nach Kristallisation aus Ethanol/Ether) 190,5-191,5°; IR (KBr): 2750, 1450, 1270, 1110 $cm^{-1}$.

Die Ausgangsverbindungen werden wie folgt hergestellt:

(a) 2-(4-Bromphenoxy)-essigsäureethylester

Analog Beispiel 6a werden 364,04 g 4-Bromphenol in die Titelverbindung überführt; Smp. (nach Kristallisation aus Hexan) 56-58°; IR ($CH_2Cl_2$): 1760, 1580, 1490, 1210 $cm^{-1}$.

(b) 3-(4-Bromphenoxy)-2-oxo-bernsteinsäurediethylester

Analog Beispiel 6b werden 356,2 g 2-(4-Bromphenoxy)-essigsäureethylester in die Titelverbindung überführt; IR ($CH_2Cl_2$): 1745, 1665, 1585, 1475, 1230 $cm^{-1}$.

(c) 5-Brom-2,3-di-(ethoxycarbonyl)-benzofuran

Analog Beispiel 6c werden 465 g 3-(4-Bromphenoxy)-2-oxo-bernsteinsäurediethylester in die Titelverbindung überführt; Smp. (nach Kristallisation aus Ether/Petrolether) 45-48°; IR ($CH_2Cl_2$): 1725, 1580, 1300 $cm^{-1}$.

(d) 5-Brom-3-ethoxycarbonyl-benzofuran

Analog Beispiel 6d werden 87,2 g 5-Brom-2,3-di-(ethoxycarbonyl)-benzofuran in die Titelverbindung überführt; Smp. (nach Kristallisation aus Petrolether) 81-83°; IR ($CH_2Cl_2$): 1725, 1560, 1440 $cm^{-1}$.

(e) 5-Brom-3-hydroxymethyl-benzofuran

Analog Beispiel 6e werden 84,5 g 5-Brom-3-ethoxycarbonyl-benzofuran in die Titelverbindung überführt; Smp. (nach Kristallisation aus Essigester/Petrolether) 72-75°; IR ($CH_2Cl_2$): 3555, 1440, 1190 $cm^{-1}$.

(f) 5-Brom-3-brommethyl-benzofuran

Analog Beispiel 6f werden 52,3 g 5-Brom-3-hydroxymethyl-benzofuran in die Titelverbindung überführt; Smp. (nach Kristallisation aus Hexan) 75-78°; IR ($CH_2Cl_2$): 1445, 1180, 1105 $cm^{-1}$.

Beispiel 11: 5-Cyano-3-(1-imidazolylmethyl)-benzofuran-hydrochlorid

Analog Beispiel 5 werden 19,2 g 5-Brom-3-(1-imidazolylmethyl)-benzofuran (s. Beispiel 10) mit 6,8 g Kupfer(I)cyanid in 60,6 ml N-Methyl-2-pyrrolidon in die Titelverbindung überführt; Smp. (nach Kristallisation aus Ethanol/Ether) 222-224°; IR: 2240, 1500, 1470 $cm^{-1}$.

Beispiel 12:

In analoger Weise wie in den Beispielen beschrieben können die folgenden Verbindungen hergestellt werden:

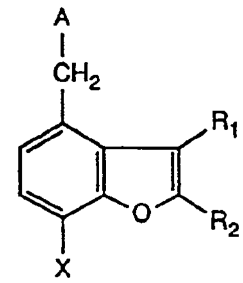

| | A | X | $R_1$ | $R_2$ | IR ($CH_2Cl_2$) [$cm^{-1}$] |
|---|---|---|---|---|---|
| (a) | 1-Imidazolyl | Cl | $CH_3$ | $CH_3$ | 1629, 1502, 1231 |
| (b) | 1-Imidazolyl | Br | H | H | 1630, 1501, 1232 |
| (c) | 1-Imidazolyl | Br | H | $CH_3$ | 1628, 1501, 1231 |
| (d) | 1-Imidazolyl | Br | $CH_3$ | H | 1630, 1500, 1231 |
| (e) | 1-Imidazolyl | Br | $-(CH_2)_4-$ | | 1630, 1503, 1230 |
| (f) | 1-Imidazolyl | Br | $C_2H_5$ | $C_2H_5$ | 1630, 1501, 1228 |
| (g) | 1-Imidazolyl | CN | H | $CH_3$ | 2248, 1625, 1504, 1234 |
| (h) | 1-Imidazolyl | CN | $CH_3$ | H | 2249, 1627, 1501, 1233 |
| (i) | 1-Imidazolyl | CN | H | H | 2247, 1628, 1500, 1235 |
| (j) | 1-Imidazolyl | CN | $-(CH_2)_4-$ | | 2250, 1629, 1502, 1231 |
| (k) | 1-(1,2,4-Triazolyl) | Cl | $CH_3$ | $CH_3$ | 1631, 1605, 1504, 1199 |
| (l) | 1-(1,2,4-Triazolyl) | Br | H | $CH_3$ | 1628, 1607, 1502, 1200 |
| (m) | 1-(1,2,4-Triazolyl) | Br | $CH_3$ | H | 1629, 1605, 1502, 1200 |
| (n) | 1-(1,2,4-Triazolyl) | Br | $-(CH_2)_4-$ | | 1630, 1604, 1504, 1198 |
| (o) | 1-(1,2,4-Triazolyl) | Br | H | H | 1631, 1605, 1503, 1199 |
| (p) | 1-(1,2,4-Triazolyl) | CN | H | H | 2233, 1615, 1504 |
| (q) | 1-(1,2,4-Triazolyl) | CN | H | $CH_3$ | 2235, 1617, 1503 |
| (r) | 1-(1,2,4-Triazolyl) | CN | $CH_3$ | H | 2232, 1615, 1503 |
| (s) | 1-(1,2,4-Triazolyl) | CN | $-(CH_2)_4-$ | | 2233, 1614, 1504 |
| (t) | 1-(1,2,4-Triazolyl) | 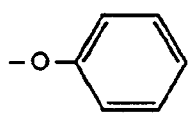 | $CH_3$ | $CH_3$ | 1602, 1505, 1227 |
| (u) | 1-(1,2,3-Triazolyl) | Br | $CH_3$ | $CH_3$ | 1631, 1605, 1504, 1199 |
| (v) | 1-(1,2,3-Triazolyl) | CN | H | $CH_3$ | 2236, 1618, 1504 |
| (w) | 1-(1,2,3-Triazolyl) | CN | $CH_3$ | H | 2235, 1616, 1506 |
| (x) | 1-(1,2,3-Triazolyl) | CN | $CH_3$ | $CH_3$ | 2236, 1617, 1505 |
| (y) | 1-(1,2,3-Triazolyl) | CN | $-(CH_2)_4-$ | | 2235, 1617, 1505 |
| (z) | 1-(1,2,3-Triazolyl) | 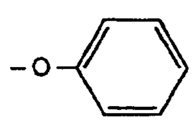 | $CH_3$ | $CH_3$ | 1602, 1506, 1228 |
| (aa) | 1-Tetrazolyl | Br | $CH_3$ | $CH_3$ | 1630, 1606, 1504, 1200 |
| (ab) | 1-Tetrazolyl | CN | H | $CH_3$ | 2234, 1616, 1505 |
| (ac) | 1-Tetrazolyl | CN | $CH_3$ | H | 2232, 1616, 1506 |
| (ad) | 1-Tetrazolyl | CN | $CH_3$ | $CH_3$ | 2232, 1618, 1505 |

| | A | X | $R_1$ | $R_2$ | IR ($CH_2Cl_2$) [$cm^{-1}$] |
|---|---|---|---|---|---|
| (ae) | 1-Tetrazolyl | CN | $-(CH_2)_4-$ | | 2235, 1617, 1503 |
| (af) | 1-Tetrazolyl | 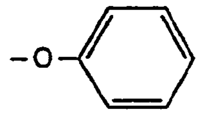 | $CH_3$ | $CH_3$ | 1602, 1505, 1225 |
| (ag) | 2-Tetrazolyl | Br | $CH_3$ | $CH_3$ | 1628, 1606, 1504, 1201 |
| (ah) | 2-Tetrazolyl | CN | H | $CH_3$ | 2234, 1616, 1505 |
| (ai) | 2-Tetrazolyl | CN | $CH_3$ | H | 2232, 1614, 1505 |
| (aj) | 2-Tetrazolyl | CN | $-(CH_2)_4-$ | | 2234, 1616, 1507 |
| (ak) | 2-Tetrazolyl | 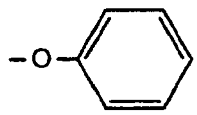 | $CH_3$ | $CH_3$ | 1602, 1507, 1227 |

Beispiel 13:

In analoger Weise wie in den vorangehenden Beispielen beschreiben können die folgenden Verbindungen hergestellt werden:

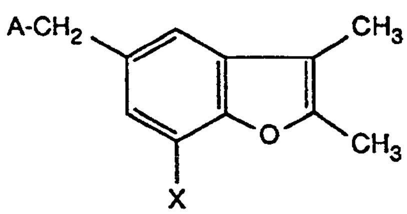

| | A | X | IR [$cm^{-1}$] |
|---|---|---|---|
| (a) | 1-(1,2,4-Triazolyl) | Br | 1635, 1604, 1507 |
| (b) | 1-(1,2,4-Triazolyl) | CN | 2238, 1617, 1505 |
| (c) | 1-Tetrazolyl | Br | 1633, 1604, 1505 |
| (d) | 1-Tetrazolyl | CN | 2237, 1619, 1504 |
| (e) | 2-Tetrazolyl | Br | 1634, 1606, 1505 |
| (f) | 2-Tetrazolyl | CN | 2236, 1618, 1504 |

Beispiel 14:

In analoger Weise wie in den vorangehenden Beispielen beschrieben können die folgenden Verbindungen hergestellt werden:

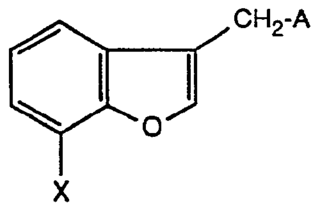

| | A | X | IR [$cm^{-1}$] |
|---|---|---|---|
| (a) | 1-Imidazolyl | 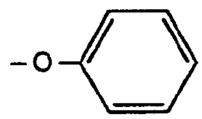 | 1602, 1506, 1495, 1227 |
| (b) | 1-(1,2,4-Triazolyl) | Br | 1632, 1605, 1504, 1198 |
| (c) | 1-(1,2,4-Triazolyl) | CN | 2236, 1618, 1505 |
| (d) | 1-(1,2,4-Triazolyl) | 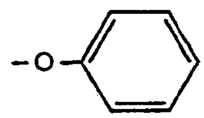 | 1601, 1507, 1496, 1228 |
| (e) | 1-Tetrazolyl | Br | 1630, 1606, 1505, 1197 |
| (f) | 1-Tetrazolyl | CN | 2235, 1615, 1505 |
| (g) | 1-Tetrazolyl | 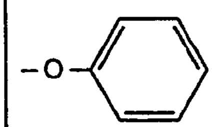 | 1600, 1507, 1494, 1227 |
| (h) | 2-Tetrazolyl | Br | 1631, 1606, 1504, 1199 |
| (i) | 2-Tetrazolyl | CN | 2235, 1617, 1508 |
| (j) | 2-Tetrazolyl | 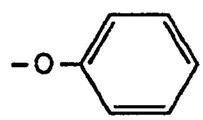 | 1602, 1506, 1495, 1228 |

Beispiel 15:

In analoger Weise wie in den vorangehenden Beispielen beschrieben können die folgenden Verbindungen hergestellt werden:

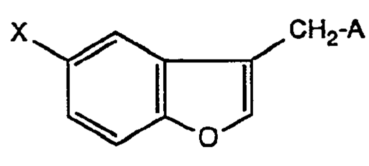

| | A | X | IR [$cm^{-1}$] |
|---|---|---|---|
| (a) | 1-(1,2,4-Triazolyl) | Br | 1632, 1606, 1503, 1198 |
| (b) | 1-(1,2,4-Triazolyl) | CN | 2237, 1614, 1502 |
| (c) | 1-(1,2,3-Triazolyl) | Br | 1630, 1605, 1502, 1200 |
| (d) | 1-(1,2,3-Triazolyl) | CN | 2234, 1615, 1504 |
| (e) | 1-Tetrazolyl | Br | 1631, 1606, 1502, 1197 |
| (f) | 1-Tetrazolyl | CN | 2235, 1615, 1503 |
| (g) | 2-Tetrazolyl | Br | 1632, 1605, 1504, 1202 |
| (h) | 2-Tetrazolyl | CN | 2237, 1614, 1504 |

Beispiel 16: (a) 7-Brom-4-(2-tetrazolylmethyl)-2,3-dimethylbenzofuran und (b) 7-Brom-4-(1-tetrazolylmethyl)-2,3-dimethylbenzofuran

Eine Lösung von 955 mg 7-Brom-4-brommethyl-2,3-dimethylbenzofuran in 15 ml Aceton wird nacheinander mit 315 mg trockenem Tetrazol, 417 mg Kaliumcarbonat und 30 mg Kaliumiodid versetzt. Nach 1,17 h Rühren bei 55° wird das Reaktionsgemisch abgekühlt und eingeengt. Der Rückstand wird in $CH_2Cl_2$/Wasser aufgenommen. Die organische Phase wird abgetrennt, mit Sole gewaschen, über Natriumsulfat getrocknet und eingeengt. Durch Säulenchromatographie ($SiO_2$, $CH_2Cl_2$) erhält man zuerst 7-Brom-4-(2-tetrazolylmethyl)-2,3-dimethylbenzofuran; Smp. (nach Umkristallisation aus Ether/Hexan): 117-120°; $^1$H-NMR (DMS0-d6): $\delta$ = 2,25 (s, 3H), 2,42 (s, 3H), 6,27 (s, 2H), 7,07 und 7,47 (Arom. H, 2H), 9,0 (s, 1H) ppm. Danach erhält man 7-Brom-4-(1-tetrazolylmethyl)-2,3-dimethylbenzofuran; Smp. (nach Umkristallisation aus Ether): 168-170°; $^1$H-NMR (DMS0-d6): $\delta$ = 2,25 (s, 3H), 2,44 (s, 3H), 6,03 (s, 2H), 6,97 und 7,47 (Arom. H, 2H), 9,47 (s, 1 H) ppm.

Beispiel 17: 7-Cyano-4-(2-tetrazolylmethyl)-2,3-dimethylbenzofuran

Eine Lösung von 154 mg 7-Brom-4-(2-tetrazolylmethyl)-2,3-dimethylbenzofuran und 50 mg Kupfer(I)cyanid in 0,9 ml N-Methyl-2-pyrrolidon wird 2,5 h bei 190-200° gerührt. Nach Abkühlen wird das Reaktionsgemisch mit $CH_2Cl_2$ verdünnt, zweimal mit wässriger Ethylendiamin-Lösung (50 %), zweimal mit Wasser und zweimal mit Sole gewaschen und nach Trocknen über Natriumsulfat eingeengt. Die Titelverbindung wird durch Säulenchromatographie ($SiO_2$, Toluol bis Toluol/Essigester 95:5) gereinigt und anschliessend aus $CH_2Cl_2$/Ether/Hexan kristallisiert; Smp. 134-135°; DC (Kieselgel, Methylenchlorid): $R_f$ = 0,3. IR ($CH_2Cl_2$); 2234, 1631, 1616, 1407 $cm^{-1}$.

Beispiel 18: 7-Carbamoyl-4-(1-imidazolylmethyl)-2,3-dimethylbenzofuran-hydrochlorid

Eine Suspension von 95 mg 7-Cyano-4-(1-imidazolylmethyl)-2,3-dimethylbenzofuranhydrochlorid in 0,5 ml DMSO und 1 ml $CH_2Cl_2$ wird bei Raumtemperatur mit 18 mg Kaliumcarbonat und 48 µl einer 30 % wässrigen $H_2O_2$-Lösung versetzt. Nach nochmaliger Zugabe von 20 mg Kaliumcarbonat und 0,2 ml $H_2O_2$-Lösung wird die Badtemperatur auf 50-60° gebracht und nach weiterer Zugabe von 0,2 ml $H_2O_2$-Lösung während 19 h bei Raumtemperatur ausgerührt. Das Reaktionsgemisch wird mit 3 ml Wasser versetzt und 1 h bei Eiskühlung gerührt Der Feststoff wird abfiltriert, mit Wasser gewaschen und im Heizexsikkator über $P_2O_5$ getrocknet. Der Feststoff wird aus Methylenchlorid/Methanol/Ether kristallisiert. Nach Lösen in Methylenchlorid/Methanol wird der Feststoff mit 40 µl 9 N methanolischer HCl-Lösung versetzt; nach Zugabe von Ether kristallisiert die Titelverbindung aus. Nach Abfiltrieren, Waschen mit Ether und Trocknen im Exsikkator erhält man die Titelverbindung. DC (Kieselgel, Methylenchlorid/Methanol 9:1) $R_f$ = 0,31; IR (KBr): 3420, 1660, 1610, 1575, 1410 $cm^{-1}$.

Beispiel 19: 7-Carbamoyl-4-[1-(1,2,4-triazolyl)methyl]-2,3-dimethylbenzofuran

Eine Lösung von 100,5 mg 7-Cyano-4-[1-(1,2,4-triazolyl)methyl]-2,3-dimethylbenzofuran (Beispiel 2) in 0,5 ml DMSO und wenig $CH_2Cl_2$ wird mit 20 mg Kaliumcarbonat und 48 µl einer 30 % Wasserstoffperoxid-Lösung versetzt. Nach 17,5 h Rühren bei Raumtemperatur werden nochmals 48 µl einer 30 % Wasserstoffperoxid-Lösung zugegeben, und es wird nochmals 3 h gerührt. Die beige Suspension wird anschliessend unter Eiskühlung mit 3 ml Wasser versetzt, 45 min gerührt und filtriert. Nach Waschen des Nutschguts wird dieses über Phosphorpentoxid getrocknet. Nach Verrühren mit $CH_2Cl_2$ erhält man die reine Titelverbindung; IR (KBr): 3427, 3188, 1694, 1616, 1503, 1407, 1272 $cm^{-1}$.

Beispiel 20: 7-N-(Cyclohexylmethyl)carbamoyl-4-[1-(1,2,4-triazolyl)methyl]-2,3-dimethylbenzofuran

Eine eisgekühlte Suspension von 136 mg 7-Carboxy-4-[1-(1,2,4-triazolyl)methyl]-2,3-dimethylbenzofuran in 4 ml $CH_2Cl_2$ und 1 ml DMF wird mit 69 µl Aminomethylcyclohexan, 114 mg N,N'-Dicyclohexylcarbodiimid, 3 mg 4-N,N-Dimethylaminopyridin und 8 mg N-Hydroxybenzotriazol versetzt. Nach 10 min wird das Kühlbad entfernt und 7 h bei Raumtemperatur gerührt. Dann wird das Reaktionsgemisch nochmals mit 57 mg N,N'-Dicyclohexylcarbodiimid, 35 µl Aminomethylcyclohexan und 2 mg 4-N,N-Dimethylaminopyridin versetzt. Nach 6 h wird der Feststoff abfiltriert. Das Filtrat wird mit $CH_2Cl_2$ verdünnt und nacheinander mit wässriger Natriumhydrogencarbonatlösung, Wasser und Sole gewaschen. Nach Trocknen und Einengen wird das Rohprodukt durch Säulenchromatographie ($SiO_2$, $CH_2Cl_2$ bis $CH_2Cl_2$/Methanol 95:5) und anschliessendes Digerieren in Hexan gereinigt; IR ($CH_2Cl_2$): 3439, 2925, 1661, 1610, 1540, 1504, 1449 $cm^{-1}$.

Die Ausgangsverbindung wird wie folgt hergestellt:

(a) 7-Carboxy-4-[1-(1,2,4-triazolyl)methyl]-2,3-dimethylbenzofuran

Eine Lösung von 252 mg 7-Cyano-4-[1-(1,2,4-triazolyl)methyl]-2,3-dimethylbenzofuran (Beispiel 2) in 4 ml Ethanol und 4 ml 4N NaOH wird 20,25 h unter Rückflussbedingungen gerührt. Nach Abkühlen durch Eiswasserkühlung wird mittels 2N $H_2SO_4$ auf pH 3 gestellt. Das Ethanol wird abgedampft und die erhaltene weisse Suspension während 1,5 h im Kühlschrank gekühlt. Das Rohprodukt wird durch Filtration erhalten und durch Verrühren in $CH_2Cl_2$ gereinigt; IR (KBr): 3429, 3140, 1693, 1610, 1512, 1405, 1292 $cm^{-1}$.

Beispiel 21: 7-N-(Cyclohexylmethyl)carbamoyl-4-(1-imidazolylmethyl)-2,3-dimethylbenzofuran

Eine Lösung von 100 mg 7-Cyano-4-(1-imidazolylmethyl)-2,3-dimethylbenzofuran (Beispiel 5) in 0,3 ml tert.-Butanol wird mit 109 mg Kaliumhydroxid versetzt und bei 80° 20 min gerührt. Nach dem Abkühlen wird das Gemisch tropfenweise mit 0,3 ml Brommethylcyclohexan versetzt und dann 30 min unter Rückfluss gerührt. Nach dem Abkühlen giesst man auf Wasser und extrahiert mit $CH_2Cl_2$. Die organische Phase wird getrocknet und eingeengt. Das Rohprodukt wird durch Säulenchromatographie ($SiO_2$, $CH_2Cl_2$ bis $CH_2Cl_2$/Methanol 95:5) gereinigt; IR ($CH_2Cl_2$): 3439, 2924, 1661, 1610, 1541, 1505, 1449, 1389 $cm^{-1}$.

Beispiel 22: 7-N-(n-Propyl)carbamoyl-4-(1-imidazolylmethyl)-2,3-dimethylbenzofuran

Analog Beispiel 21 werden 100 mg 7-Cyano-4-(1-imidazolylmethyl)-2,3-dimethylbenzofuran in 0,3 ml tert.-Butanol mit 109 mg Kaliumhydroxid und 178 µl n-Propylbromid in die Titelverbindung überführt. Diese wird durch Säulenchromatographie ($SiO_2$, $CH_2Cl_2$ bis $CH_2Cl_2$/Methanol 98:2) und Verrühren in Hexan gereinigt; IR ($CH_2Cl_2$): 3435, 3040, 1660, 1610, 1539, 1505, 1457, 1389 $cm^{-1}$.

Beispiel 23: 7-N-(2-Propyl)carbamoyl-4-(1-imidazolylmethyl)-2,3-dimethylbenzofuran

Analog Beispiel 21 werden 135 mg 7-Cyano-4-(1-imidazolylmethyl)-2,3-dimethylbenzofuran in 0,4 ml tert.-Butanol mit 148 mg Kaliumhydroxid und total 515 µl Isopropyliodid innerhalb von 8 h in die Titelverbindung überführt. Diese wird durch Säulenchromatographie ($SiO_2$, $CH_2Cl_2$ bis $CH_2Cl_2$/Methanol 98:2) gereinigt; IR ($CH_2Cl_2$): 4325, 2966, 1658, 1610, 1535, 1505, 1457, 1387 $cm^{-1}$.

Beispiel 24:

Es werden 10000 Tabletten hergestellt, die je 5 mg Wirkstoff, z.B. eine der in den Beispielen 1-23 hergestellten Verbindungen, enthalten:

| Zusammensetzung: | |
|---|---|
| Wirkstoff | 50,00 g |
| Milchzucker | 2535,00 g |
| Maisstärke | 125,00 g |
| Polyethylenglykol 6.000 | 150,00 g |
| Magensiumstearat | 40,00 g |
| Gereinigtes Wasser | quantum satis |

Verfahren:

Alle pulverigen Bestandteile werden durch ein Sieb mit einer Maschenweite von 0,6 mm gesiebt. Dann wird der Wirkstoff, der Milchzucker, das Magnesiumstearat und die Hälfte der Stärke in einem geeigneten Mischer vermischt. Die andere Hälfte der Stärke wird in 65 ml Wasser suspendiert und die entstandene Suspension zu einer siedenden Lösung des Polyethylenglykols in 260 ml Wasser gegeben. Die gebildete Paste wird dem Pulvergemisch zugesetzt und gegebenenfalls unter Zugabe von mehr Wasser granuliert. Das Granulat wird über Nacht bei 35°C getrocknet, durch ein Sieb mit 1,2 mm Maschenweite getrieben und zu Tabletten, welche eine Bruchrille aufweisen, gepresst.

Beispiel 25:

Es werden 1000 Kapseln hergestellt, die je 10 mg Wirkstoff, z.B. eine der in den Beispielen 1-23 hergestellten Verbindungen, enthalten:

| Zusammensetzung: | |
|---|---|
| Wirkstoff | 10,00 g |
| Milchzucker | 207,00 g |
| Modifizierte Stärke | 80,00 g |
| Magnesiumstearat | 3,00 g |

Verfahren:

Alle pulverigen Bestandteile werden durch ein Sieb mit einer Maschenweite von 0,6 mm gesiebt. Dann wird der Wirkstoff in einem geeigneten Mischer zuerst mit dem Magensiumstearat und dann mit dem Milchzucker und der Stärke bis zur Homogenität vermischt. Hartgelatinekapseln Nr. 2 werden mit je 300 mg der erhaltenen Mischung unter Verwendung einer Kapselfüllmaschine gefüllt.

## Patentansprüche

1. Verbindungen der Formel I

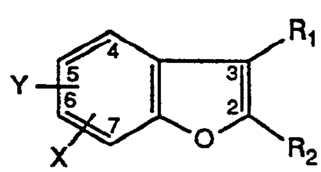

(I)

worin X Halogen, Cyano, Carbamoyl, N-Niederalkylcarbamoyl, N,N-Diniederalkylcarbamoyl, N,N-Niederalkylencarbamoyl; durch -O-, -S- oder -NR″- unterbrochenes N,N-Niederalkylencarbamoyl, worin R″ für Wasserstoff, Niederalkyl oder Niederalkanoyl steht; N-Cycloalkylcarbamoyl, N-(Niederalkyl-substituiertes

Cycloalkyl)-carbamoyl, N-Cycloakylniederakylcarbamoyl, N-(Niederalkyl-substituiertes Cycloakyl)-niederalkylcarbamoyl, N-Arylniederalkylcarbamoyl, N-Arylcarbamoyl, N-Hydroxycarbamoyl, Hydroxy, Niederalkoxy, Arylniederalkoxy oder Aryloxy bedeutet, Y für eine Gruppe $-CH_2-A$ steht, in der A über ein Ringstickstoffatom gebundenes Imidazolyl, Triazolyl oder Tetrazolyl bedeutet, oder Y Wasserstoff bedeutet, $R_1$ und $R_2$ unabhängig voneinander Wasserstoff, Niederalkyl oder eine Gruppe $-CH_2-A$ wie für Y definiert bedeuten, oder $R_1$ und $R_2$ zusammen Niederalkylen bedeuten, mit der Massgabe, dass einer der Reste Y, $R_1$ oder $R_2$ für eine Gruppe $-CH_2-A$ steht, mit der weiteren Massgabe, dass in einer Gruppe $-CH_2-A$ als Bedeutung von $R_1$ oder $R_2$ A von 1-Imidazolyl verschieden ist, wenn X Brom, Cyano oder Carbamoyl bedeutet, und mit der Massgabe, dass in einer Gruppe $-CH_2-A$ als Bedeutung von Y A von 1-Imidazolyl verschieden ist, wenn X Halogen oder Niederalkoxy bedeutet, $R_1$ für Wasserstoff steht und $R_2$ Wasserstoff oder Niederalkyl bedeutet, und Salze davon.

2. Verbindungen der Formel I gemäss Anspruch 1, worin X Halogen, Cyano, Carbamoyl, N-Niederalkylcarbamoyl, N-Cycloalkylniederalkylcarbamoyl, N,N-Diniederalkylcarbamoyl, N-Arylcarbamoyl, Hydroxy, Niederalkoxy, Arylniederalkoxy oder Aryloxy bedeutet, wobei Aryl für Phenyl oder Naphthyl, welches jeweils unsubstituiert oder durch Niederaklyl, Hydroxy, Niederalkoxy, Halogen und/oder Trifluormethyl substituiert ist, steht; Y für eine Gruppe $-CH_2-A$ steht, in der A 1-Imidazolyl, 1-(1,2,4-Triazolyl), 1-(1,3,4-Triazolyl), 1-(1,2,3-Triazolyl), 1-(1,2,5-Triazolyl), 1-Tetrazolyl oder 2-Tetrazolyl bedeutet, oder Y Wasserstoff bedeutet, $R_1$ und $R_2$ unabhängig voneinander Wasserstoff, Niederalkyl oder eine Gruppe $-CH_2-A$ wie für Y definiert bedeuten, oder $R_1$ und $R_2$ zusammen $-(CH_2)_n-$, worin n für 3, 4 oder 5 steht, bedeuten, mit der Massgabe, dass einer der Reste Y, $R_1$ oder $R_2$ für eine Gruppe $-CH_2-A$ steht, mit der weiteren Massgabe, dass in einer Gruppe $-CH_2-A$ als Bedeutung von $R_1$ oder $R_2$ A von 1-Imidazolyl verschieden ist, wenn X Brom, Cyano oder Carbamoyl bedeutet, und mit der Massgabe, dass in einer Gruppe $-CH_2-A$ als Bedeutung von Y A von 1-Imidazolyl verschieden ist, wenn X Halogen oder Niederalkoxy bedeutet, $R_1$ für Wasserstoff steht und $R_2$ Wasserstoff oder Niederalkyl bedeutet, und Salze davon.

3. Verbindungen der Formel I gemäss Anspruch 1, worin der Rest X in 5- oder 7-Stellung angeknüpft ist und Halogen, Cyano, Carbamoyl oder Phenyloxy bedeutet; der Rest Y in 4-oder 5-Stellung angeknüpft ist und für eine Gruppe $-CH_2-A$ steht, in der A 1-Imidazolyl, 1-(1,2,4-Triazolyl), 1-(1,2,3-Triazolyl), 1-Tetrazolyl oder 2-Tetrazolyl bedeutet, oder der Rest Y Wasserstoff bedeutet; $R_1$ Wasserstoff, Niederalkyl oder eine Gruppe $-CH_2-A$ wie für Y definiert bedeutet, $R_2$ Wasserstoff oder Niederalkyl bedeutet, oder $R_1$ und $R_2$ zusammen $-(CH_2)_4-$ bedeuten; mit der Massgabe, dass einer der Reste Y oder $R_1$ für eine Gruppe $-CH_2-A$ steht; mit der weiteren Massgabe, dass in einer Gruppe $-CH_2-A$ als Bedeutung von $R_1$ A von 1-Imidazolyl verschieden ist, wenn X Brom, Cyano oder Carbamoyl bedeutet, und mit der Massgabe, dass in einer Gruppe $-CH_2-A$ als Bedeutung von Y A von 1-Imidazolyl verschieden ist, wenn X Halogen bedeutet und $R_1$ für Wasserstoff steht; und Salze davon.

4. Verbindungen der Formel I gemäss Anspruch 1, worin der Rest X in 5- oder 7-Stellung angeknüpft ist und Halogen, Cyano, Carbamoyl oder Phenyloxy bedeutet; der Rest Y in 4- oder 5-Stellung angeknüpft ist und für eine Gruppe $-CH_2-A$ steht, in der A 1-(1,2,4-Triazolyl), 1-(1,2,3-Triazolyl), 1-Tetrazolyl oder 2-Tetrazolyl bedeutet, oder der Rest Y Wasserstoff bedeutet; $R_1$ Wasserstoff, Niederalkyl oder eine Gruppe $-CH_2-A$ wie für Y definiert bedeutet, $R_2$ Wasserstoff oder Niederalkyl bedeutet, oder $R_1$ und $R_2$ zusammen $-(CH_2)_4-$ bedeuten; mit der Massgabe, dass einer der Reste Y oder $R_1$ für eine Gruppe $-CH_2-A$ steht; und Salze davon.

5. Verbindungen der Formel I gemäss Anspruch 1, worin der Rest X in 5- oder 7-Stellung angeknüpft ist und Halogen, Cyano, Carbamoyl oder Phenyloxy bedeutet; der Rest Y in 4-oder 5-Stellung angeknüpft ist und für eine Gruppe $-CH_2-A$ steht, in der A 1-Imidazolyl, 1-(1,2,4-Triazolyl), 1-(1,2,3-Triazolyl), 1-Tetrazolyl oder 2-Tetrazolyl bedeutet; $R_1$ und $R_2$ unabhängig voneinander Wasserstoff oder Niederalkyl bedeuten, oder $R_1$ und $R_2$ zusammen für $-(CH_2)_4-$ stehen; mit der Massgabe, dass in einer Gruppe $Y = -CH_2-A$ A von 1-Imidazolyl verschieden ist, wenn X Halogen bedeutet und $R_1$ für Wasserstoff steht; und Salze davon.

6. Verbindungen der Formel I gemäss Anspruch 1, worin der Rest X in 5- oder 7-Stellung angeknüpft ist und Halogen, Cyano, Carbamoyl oder Phenyloxy bedeutet; der Rest Y Wasserstoff bedeutet; $R_1$ für eine Gruppe $-CH_2-A$ steht, in der A 1-Imidazolyl, 1-(1,2,4-Triazolyl), 1-(1,2,3-Triazolyl), 1-Tetrazolyl oder 2-Tetrazolyl bedeutet, $R_2$ Wasserstoff oder Niederalkyl bedeutet, mit der Massgabe, dass in einer Gruppe $R_1 = -CH_2-A$ A von 1-Imidazolyl verschieden ist, wenn X Brom, Cyano oder Carbamoyl bedeutet; und Salze davon.

7. Verbindungen der Formel I gemäss Anspruch 1, worin der Rest X in 7-Stellung angeknüpft ist und Brom, Cyano, Carbamoyl oder Phenyloxy bedeutet; der Rest Y in 4-Stellung angeknüpft ist und für eine Gruppe -$CH_2$-A steht, in der A 1-Imidazolyl, 1-(1,2,4-Triazolyl), 1-Tetrazolyl oder 2-Tetrazolyl bedeutet; $R_1$ und $R_2$ unabhängig voneinander Niederalkyl bedeuten, oder $R_1$ und $R_2$ zusammen für -$(CH_2)_4$- stehen; und Salze davon.

8. Verbindungen der Formel I gemäss Anspruch 1, worin X Halogen, Cyano, Carbamoyl, Hydroxy, Niederalkoxy oder Phenyloxy, worin Phenyl unsubstituiert oder durch Niederalkyl, Hydroxy, Niederalkoxy, Halogen und/oder Trifluormethyl substituiert ist, bedeutet, Y für eine Gruppe -$CH_2$-A steht, in der A 1-Imidazolyl, 1-(1,2,4-Triazolyl), 1-(1,3,4-Triazolyl), 1-(1,2,3-Triazolyl), 1-(1,2,5-Triazolyl), 1-Tetrazolyl oder 2-Tetrazolyl bedeutet, $R_1$ Niederalkyl bedeutet, $R_2$ Wasserstoff oder Niederalkyl bedeutet, oder $R_1$ und $R_2$ zusammen -$(CH_2)_n$-, worin n für 3 oder 4 steht, bedeuten, und Salze davon.

9. Verbindungen der Formel I gemäss Anspruch 1, worin der Rest X in 4- oder 7-Stellung angeknüpft ist und Halogen, Cyano, Carbamoyl oder Phenyloxy bedeutet; der Rest Y in 4- oder 5-Stellung angeknüpft ist und für eine Gruppe -$CH_2$-A steht, in der A 1-Imidazolyl, 1-(1,2,4-Triazolyl), 1-(1,2,3-Triazolyl), 1-Tetrazolyl oder 2-Tetrazolyl bedeutet, $R_1$ Niederalkyl bedeutet, $R_2$ Wasserstoff oder Niederalkyl bedeutet, oder $R_1$ und $R_2$ zusammen -$(CH_2)_4$- bedeuten; und Salze davon.

10. 7-Cyano-4-[1-(1,2,4-triazolyl)methyl]-2,3-dimethylbenzofuran gemäss Anspruch 1, oder ein pharmazeutisch anwendbares Salz davon.

11. 7-Cyano-4-(1-imidazolylmethyl)-2,3-dimethylbenzofuran gemäss Anspruch 1, oder ein pharmazeutisch anwendbares Salz davon.

12. 7-Carbamoyl-4-(1-imidazolylmethyl)-2,3-dimethylbenzofuran gemäss Anspruch 1, oder ein pharmazeutisch anwendbares Salz davon.

13. Pharmzeutische Präparate enthaltend eine Verbindung gemäss einem der Ansprüche 1 bis 12 und mindestens ein pharmazeutisch verwendbares Trägermaterial.

14. Eine Verbindung gemäss einem der Ansprüche 1 bis 12 zur Anwendung in einem Verfahren zur therapeutischen Behandlung des tierischen oder menschlichen Körpers.

15. Eine Verbindung gemäss einem der Ansprüche 1 bis 12 zur Verwendung bei der Behandlung von Krankheiten, die auf eine Hemmung des Enzyms Aromatase ansprechen.

16. Verwendung einer Verbindung gemäss einem der Ansprüche 1 bis 12 zur Herstellung pharmazeutischer Präparate.

17. Verwendung einer Verbindung gemäss einem der Ansprüche 1 bis 12 zur Herstellung pharmazeutischer Präparate für die Behandlung von Krankheiten, die auf eine Hemmung des Enzyms Aromatase ansprechen.

18. Verfahren zur Herstellung einer Verbindung der Formel I gemäss Anspruch 1, dadurch gekennzeichnet, dass man

(a) ein reaktives verestertes Derivat einer Hydroxymethyl-Verbindung der Formel II

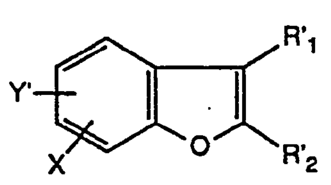

(II)

worin einer der Reste Y', $R'_1$ oder $R'_2$ Hydroxymethyl bedeutet und die beiden anderen Reste jeweils wie Y, $R_1$ bzw. $R_2$ unter Formel I definiert sind, und X wie unter Formel I definiert ist, mit einer Verbindung

A - H (III)

worin A wie unter Formel I definiert ist, oder einem N-geschützten Derivat davon, kondensiert, oder (b) in einer Verbindung der Formel IV

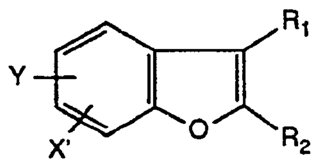

(IV)

worin X' einen in eine Gruppe X überführbaren Rest bedeutet und Y, $R_1$ und $R_2$ wie unter Formel I definiert sind, den Rest X' in eine Gruppe X überführt, oder

(c) zur Herstellung von Verbindungen der Formel I, worin A in der Gruppe $-CH_2-A$ 1-Tetrazolyl bedeutet, eine Verbindung der Formel V

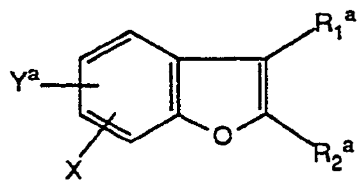

(V)

worin einer der Reste $Y^a$, $R_1{}^a$ oder $R_2{}^a$ Isocyanomethyl bedeutet und die beiden anderen Reste jeweils wie Y, $R_1$ bzw. $R_2$ unter Formel I definiert sind, und X wie unter Formel I definiert ist, mit Stickstoffwasserstoffsäure oder insbesondere einem Salz davon umsetzt; und/oder, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, und/oder ein erhaltenes Gemisch von isomeren Verbindungen der Formel I in die einzelnen Isomeren auftrennt.

Europäisches Patentamt

# EUROPÄISCHER RECHERCHENBERICHT

Nummer der Anmeldung

EP 91 81 0110

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 316 097 (ICI)<br>* Zusammenfassung; Ansprüche 1,8 *<br>--- | 1,2,15, 17,18 | C 07 D 405/06<br>A 61 K 31/41 |
| Y | EP-A-0 293 978 (JANSSEN PHARMACEUTICA N.V.)<br>* Zusammenfassung; Ansprüche 1,7,10,11I *<br>--- | 1,2,15, 17,18 | |
| Y | EP-A-0 073 663 (PFIZER CORPORATION)<br>* Ansprüche 1,3,4; Seite 70, Beispiel 16IV; Zusammenfassung *<br>--- | 1,2,15, 17,18 | |
| A | EP-A-0 337 928 (SCHERING AG)<br>* Zusammenfassung; Seite 3, Zeilen 14-20; Ansprüche 1-4 *<br>--- | 1,15 | |
| A | EP-A-0 165 783 (ELI LILLY & CO.)<br>* Anspruch 1; Seite 9, Beispiel *<br>--- | 1,15 | |
| A | EP-A-0 296 749 (ICI)<br>* Zusammenfassung; Anspruch 1 *<br>----- | 1,15 | |

| RECHERCHIERTE SACHGEBIETE (Int. Cl.5) |
|---|
| C 07 D 405/00 |

Der vorliegende Recherchenbericht wurde für alle Patentansprüche erstellt

| Recherchenort | Abschlußdatum der Recherche | Prüfer |
|---|---|---|
| DEN HAAG | 29-05-1991 | PAISDOR B. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)